## Europäisches Patentamt

## European Patent Office

## Office européen des brevets

(11) Veröffentlichungsnummer: **0 448 799 A2**

# EUROPÄISCHE PATENTANMELDUNG

(21) Anmeldenummer: 90123843.6

(22) Anmeldetag: 11.12.90

(51) Int. Cl.⁵: **C07H 15/04**

(30) Priorität: 28.02.90 DE 4006192

(43) Veröffentlichungstag der Anmeldung:
02.10.91 Patentblatt 91/40

(84) Benannte Vertragsstaaten:
**BE DE ES FR GB IT NL**

(71) Anmelder: **HÜLS AKTIENGESELLSCHAFT**
**Patentabteilung / PB 15 - Postfach 13 20**
**W-4370 Marl 1(DE)**

(72) Erfinder: **Ripke, Norbert, Dr.**
**Hellweg 28**
**W-4358 Haltern(DE)**

(54) Verfahren zur Herstellung von Kohlenhydrattensiden.

(57)
1. Verfahren zur Herstellung von Kohlenhydrattensiden

2.1 Übliche Verfahren zur Herstellung von Alkylpolyglycosiden mit Tensidcharakter sind zwei- oder mehrstufig. In einstufiger Reaktion gelingt die Umsetzung von polarem Saccharid und unpolarem langkettigem Alkohol bisher nur bei speziellen Saccharid-Alkohol-Mengenverhältnissen sowie bei kontrollierter Katalyse unter langsamer Wasserentwicklung.

2.2 In einem unpolaren Lösemittel und unter Zusatz eines Emulgators wird nun die Herstellung von Kohlenhydrattensiden einstufig bei großer Variationsmöglichkeit hinsichtlich der Mengenverhältnisse, der Katalyse und der Wasserentwicklung durchgeführt.

2.3 Herstellung von Kohlenhydrattensiden und Tensidmischungen.

Verfahren zur Herstellung von Kohlenhydrattensiden

Die Erfindung betrifft ein Verfahren zur einstufigen Herstellung von Alkylpolyglycosiden mit Tensidcharakter.

Alkylpolyglycoside sind ungiftige und leicht abbaubare oberflächenaktive Stoffe. Sie werden deshalb als Wasch- und Reinigungsmittel und als Emulgatoren und Dispergatoren verwendet. Sie haben aber nur dann die gewünschten Grenzflächeneigenschaften, wenn die Alkylgruppen mindestens 8 C-Atome aufweisen.

Alkylglycoside und Alkylpolyglycoside mit langkettigen Alkylgruppen werden im allgemeinen durch mehrstufige Synthesen hergestellt. So wird nach US 3 219 656 zur Herstellung der Alkylglucoside ein doppelter Alkoholaustausch vorgenommen. Danach wird Glucose zuerst in Methylglucosid, dann in Butylglucosid und danach in das gewünschte langkettige Alkylglucosid übergeführt.

Ein einfacheres zweistufiges Verfahren wird beispielsweise in EP-A-0 306 652 angegeben, wonach man zunächst durch Glycosidierung mit n-Butanol ein n-Butylglycosid und daraus durch Umglycosidierung mit einem langkettigen Alkohol das gewünschte langkettige Alkylpolyglycosid herstellt.

Nach US 3 839 318 kann auch eine einstufige Reaktion zur Herstellung von Alkyloligosacchariden durchgeführt werden. Dabei müssen die Reaktionsbedingungen jedoch sorgfältig eingehalten werden. Das Glucose-Alkohol-Verhältnis muß in sehr engen Grenzen gehalten werden. Vor allem aber ist es erforderlich, daß die Wasserbildung langsam verläuft und genau kontrolliert wird. Das Wasser muß schneller entfernt werden als es gebildet wird. Zur Abtrennung des Wassers kann eine Vakuumdestillation oder eine Azeotropdestillation mit kleinen Mengen an Kohlenwasserstoffen durchgeführt werden. - Nach diesem Verfahren werden nur bei Einhaltung der optimalen Bedingungen gute Umsätze erzielt.

Gemäß US-H-619 kann man eine Umsetzung von Sacchariden mit langkettigen Alkoholen auch unter Zusatz von 1 bis 25 Molen N-Methyl-2-pyrrolidon pro Mol Saccharid durchführen. Das polare N-Methyl-2-pyrrolidon hat einen hohen Siedepunkt von 202 °C und ist deshalb destillativ schwer zu entfernen. Außerdem können nur Alkylglycoside hergestellt werden, da N-Methyl-2-pyrrolidon die Polymerisation zu den Alkylpolyglycosiden verhindert.

Es bestand daher die Aufgabe, ein gutmütiges einstufiges Verfahren zur Herstellung von Alkylpolyglycosiden mit langkettigen Alkylgruppen bereitzustellen.

Die Aufgabe wird erfindungsgemäß dadurch gelöst, daß man die Saccharide und die langkettigen Alkohole in einem unpolaren Lösemittel in Gegenwart von Emulgatoren umsetzt.

Als Saccharide können Monosaccharide, wie Glucose, Mannose, Gulose, Galaktose oder Talose, aber auch Di- und Oligosaccharide mit bis zu 10 Saccharideinheiten verwendet werden. Die Einheiten können 1,2-, 1,3-, 1,4- oder 1,6-verknüpft sein. Es können α- oder β-Verknüpfungen vorliegen. Die Ketten können linear oder verzweigt sein. Vorzugsweise wird Glucose eingesetzt.

Die eingesetzten Alkohole weisen 8 bis 24 C-Atome auf. Vorzugsweise enthalten sie 10 bis 18 C-Atome. Die Alkohole können linear sein. Sie können aber auch Verzweigungen enthalten. Sie können gesättigt sein oder auch olefinische Doppelbindungen enthalten. Man kann natürliche oder synthetische Fettalkohole oder Fettalkoholgemische einsetzen. Als Beispiele seien Decanol, 10-Undecen-1-ol, Dodecanol, Myristylalkohol und Stearylalkohol genannt.

Die Herstellung der Alkylpolyglycoside aus Sacchariden und Alkoholen wird durch Säuren katalysiert. Man kann Mineralsäuren, wie Schwefel- oder Phosphorsäure, einsetzen. Gut geeignet sind auch organische Säuren, wie beispielsweise Aryl-, Alkyl- oder Aralkylsulfonsäuren.

Die hergestellten Alkylpolyglycoside weisen einen mittleren Polymerisationsgrad von 1 bis 10 auf. Dabei werden niedrige mittlere Polymerisationsgrade von 1,3 bis 5 bevorzugt.

Als Lösemittel sind gesättigte, ungesättigte, lineare oder verzweigte Kohlenwasserstoffe, Ether, Ketone und Halogenkohlenwasserstoff geeignet. Dabei können auch Lösemittelgemische verwendet werden.

Bevorzugt sind Kohlenwasserstoffe, wie beispielsweise Pentan, Hexan, Cyclohexan, Octan, Decan, oder Aromaten, wie Benzol, Toluol oder Xylol.

Außerdem sind auch Ether bevorzugt. Beispiele dafür sind Diisopropylether, Dibutylether, Methyl-tert.-butylether, Tetrahydrofuran oder Dioxan.

Dabei sind Lösemittel mit einem Siedepunkt im Bereich von 50 bis 180 °C besonders bevorzugt.

Vorzugsweise werden aus den Sacchariden und den Alkoholen mit den unpolaren Lösemitteln 30- bis 75%ige Lösungen hergestellt.

In diese Lösungen werden außerdem vorzugsweise 4 bis 100 % Emulgator, bezogen auf die Reaktionskomponenten, gegeben. Als Emulgatoren werden gebräuchliche nichtionische, anionische oder kationische Tenside und andere grenzflächenaktive Verbindungen eingesetzt. Man kann auch Kombinationen dieser Substanzen verwenden.

Beispiele für geeignete Emulgatoren sind Saccharosefettsäureester, Sorbitanfettsäureester und Fettalkoholethoxylate.

Bevorzugt werden Paraffinsulfonate, Alkylpolyglycoside mit Alkylgruppen mit 8 bis 24 C-Atomen und andere Kohlenhydrattenside verwendet.

Die Reaktion wird vorzugsweise bei 60 bis 160 °C durchgeführt. Dabei wird ein Temperaturbereich von 80 bis 120 °C besonders bevorzugt. Die Reaktion kann bei Normaldruck, bei leichtem Unterdruck und auch bei Überdruck ausgeführt werden.

Nach dem erfindungsgemäßen Verfahren wird allgemein so vorgegangen, daß man Saccharid, Alkohol, Katalysator, Lösemittel und Emulgator mischt und auf die Reaktionstemperatur erhitzt. Dabei wird Wasser gegebenenfalls azeotrop destilliert. Wenn die Wasserdestillation beendet ist, werden Lösemittel und überschüssiger Alkohol destilliert. Der Emulgator kann gegebenenfalls ebenfalls destilliert werden. Man kann den Emulgator auch im Produkt belassen, so daß dann eine Kohlenhydrattensid-Emulgator-Mischung weiterverwendet wird. Produkt oder Mischung werden nach bekannten Methoden gereinigt und aufgearbeitet.

Emulgator und Lösemittel ermöglichen einen guten Kontakt zwischen den hydrophilen Kohlenhydraten und den hydrophoben langkettigen Alkoholen.

Das vorliegende einstufige Verfahren ist gegenüber den mehrstufigen Verfahren technisch einfacher. Es erlaubt darüber hinaus eine große Bandbreite hinsichtlich des Alkohol-Saccharid-Verhältnisses und des einzusetzenden Katalysators. Das Verfahren liefert die gewünschten Produkte bei guten Umsätzen in hohen Ausbeuten und guter Reinheit.

Die folgenden Beispiele sollen die Erfindung verdeutlichen.

Beispiel 1

In einem 500-ml-Rührkessel mit aufgesetzter Kolonne und Wasserabscheider werden 50 g Paraffinsulfonsäure (MARLON[R] PS, Fa. Hüls AG, D-4370 Marl) 10 g Glucose (56 mMol), 50 g eines Gemisches aus 85 % Dodecanol und 15 % Tetradecanol (261 mMol) und 100 g Diisopropylether auf 120 °C bei Normaldruck erhitzt. Das bei der Reaktion gebildete Wasser siedet azeotrop und wird im Wasserabscheider abgetrennt. Nach 15 Minuten hat sich 1 g Wasser gebildet, worauf die Reaktion durch Abkühlung auf Raumtemperatur beendet wird.

Die Lösung wird mit 1 n wäßriger Natronlauge auf pH 7 gestellt, worauf Wasser, Diisopropylether und danach der Überschuß an Alkoholmischung destilliert werden. Das resultierende Produkt enthält weniger als 0,3 % Glucose.

Ausbeute:　　14,7 g Dodecyl-Tetradecylpolyglucosid 85 - 15 (93 % d. Th.) (mittlerer Polymerisationsgrad: 1,6)

In diesem Beispiel dient der Emulgator auch als Katalysator.

Beispiel 2

In einem 1 000-ml-Rührkessel mit aufgesetzter Kolonne und Wasserabscheider werden 345 g eines Gemisches aus 85 % Dodecanol und 15 % Tetradecanol, 80 g Dodecyl-Tetradecylpolyglucosid 85 - 15 (Polymerisationsgrad 1,7) und 200 g Cyclohexan vorgelegt. Die Säurezahl des Gemisches wird mit p-Toluolsulfonsäure auf 2,0 mg KOH/g eingestellt. Diese Säurezahl wird während der Reaktion konstant gehalten.

Man erwärmt auf 121 °C, worauf man kontinuierlich pro Stunde 21 g Glucose und 84 g eines Gemisches aus 85 % Dodecanol und 15 % Tetradecanol zusetzt. Der Füllstand des Reaktors wird durch kontinuierliche Entnahme des Reaktionsprodukts konstant gehalten. Lösemittel und Katalysator werden in dem Maße, wie sie mit der Reaktionsmischung entnommen werden, nachdosiert. Gleichmäßige Reaktionsbedingungen werden so aufrechterhalten.

Die Reaktionsmischung wird mit 1 n wäßriger NaOH auf pH 9 eingestellt. Dann werden Cyclohexan, Wasser und das restliche Alkoholgemisch über zwei Destillationsstufen abdestilliert. Cyclohexan und Alkoholgemisch werden in die Reaktion wieder eingesetzt.

Als Destillationsrückstand erhält man ein Reaktionsprodukt, das weniger als 0,3 % Glucose aufweist: 30,5 g/h Dodecyl-Tetradecylpolyglucosid 85 - 15 (97 % d. Th.) (mittlerer Polymerisationsgrad: 1,8). Während der Reaktion braucht der Emulgator, das Alkylpolyglucosid, nicht nachdosiert zu werden, da er als Reaktionsprodukt kontinuierlich nachgebildet wird.

**Patentansprüche**

.

1.  Verfahren zur Herstellung von Alkylpolyglycosiden mit Alkylgruppen mit 8 bis 24 C-Atomen
    und einem mittleren Polymerisationsgrad von 1
    bis 10 aus Sacchariden und Alkoholen mit 8
    bis 24 C-Atomen in einstufiger Reaktion,
    dadurch gekennzeichnet,
    daß man die Saccharide mit den Alkoholen in
    einem unpolaren Lösemittel in Gegenwart von
    Emulgatoren umsetzt.

2.  Verfahren nach Anspruch 1,
    dadurch gekennzeichnet,
    daß man Kohlenwasserstoffe und/oder Ether
    als Lösemittel einsetzt.

3.  Verfahren nach Anspruch 1,
    dadurch gekennzeichnet,
    daß man Lösemittel mit Siedepunkten von 50
    bis 180 °C einsetzt.

4.  Verfahren nach Anspruch 1,
    dadurch gekennzeichnet,
    daß 4 bis 100 % Emulgator, bezogen auf die
    Reaktionskomponenten, verwendet werden.

5.  Verfahren nach Anspruch 1,
    dadurch gekennzeichnet,
    daß ein Alkylpolyglycosid mit einer Alkylgruppe mit 8 bis 24 C-Atomen als Emulgator eingesetzt wird.

6.  Verfahren nach Anspruch 1,
    dadurch gekennzeichnet,
    daß man die Reaktion bei 60 bis 160 °C,
    vorzugsweise bei 80 bis 120 °C, durchführt.

7.  Verfahren nach Anspruch 1,
    dadurch gekennzeichnet,
    daß man Alkylpolyglycoside mit einem mittleren Polymerisationsgrad von 1,3 bis 5 herstellt.

8.  Verfahren nach Anspruch 1,
    dadurch gekennzeichnet,
    daß Alkohole mit 10 bis 18 C-Atomen eingesetzt werden.

9.  Verfahren nach Anspruch 1,
    dadurch gekennzeichnet,
    daß man 30- bis 70%ige Lösungen der Saccharide und Alkohole in dem unpolaren Löse-
    mittel einsetzt.